# EUROPEAN PATENT APPLICATION

(11) **EP 2 116 547 A1**
(43) Date of publication of application: **11.11.2009**
(21) Application number: 08380146.4
(22) Date of filing: 09.05.2008
(51) Int. Cl.: C07D 513/04, C07D 519/00, A61K 31/429, A61P 3/04, A61P 25/00, A61P 25/16, A61P 25/28

(54) **Substituted N-imidazo(2, 1-b) thiazole-5-sulfonamide derivatives as 5-TH6 ligands**

(71) Applicant: Laboratorios Del. Dr. Esteve, S.A., 08041 Barcelona (ES)
(72) Inventor: Mas Prió, Josep, E-08191 Rubi-Barcelona (ES); Torrens Jover, Antoni, E-08221 Terrasa-Barcelona (ES)
(74) Representative: ABG Patentes, S.L.

(57) **Abstract**

The invention relates to compounds (I) having pharmacological activity towards the 5-HT₆ receptor, and more particularly to some *N*-imidazo[2,1-b]thiazole-5-sulfonamide derivatives, to processes of preparation of such compounds, to pharmaceutical compositions comprising them, and to their use for the treatment and or prophylaxis of a disease in which 5-HT₆ is involved.

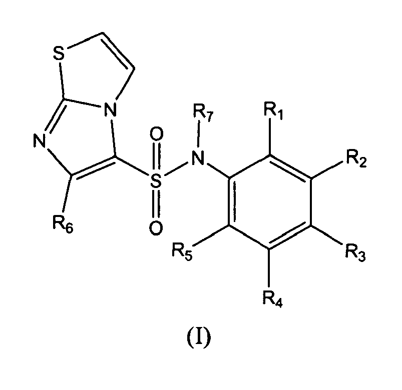

## Description

### TECHNICAL FIELD

The present invention relates to compounds having pharmacological activity towards the 5-HT₆ receptor, and more particularly to substituted *N*-imidazo[2,1-b]thiazole-5-sulfonamide compounds, to processes of preparation of such compounds, to pharmaceutical compositions comprising them, and to their use in therapy, in particular for the treatment and/or prophylaxis of a disorder or a disease in which 5-HT₆ is involved.

### BACKGROUND OF THE INVENTION

The search for new therapeutic agents has been greatly aided in recent years by better understanding of the structure of proteins and other biomolecules associated with target diseases. One important class of proteins that has been the subject of extensive study is the family of 5-hydroxytryptamine (serotonin, 5-HT) receptors. The superfamily of serotonin receptors (5-HT) includes 7 classes (5-HT₁-5-HT₇) encompassing 14 human subclasses [D. Hoyer, et al., Neuropharmacology, 1997, 36, 419]. The 5-HT₆ receptor is the latest serotonin receptor identified by molecular cloning both in rats [F.J. Monsma, et al., Mol. Pharmacol., 1993, 43, 320; M. Ruat, et al., Biochem. Biophys. Res. Commun., 1993, 193, 268] and in humans [R. Kohen, et al., J. Neurochem., 1996, 66, 47]. Compounds with 5-HT₆ receptor affinity are useful for the treatment of various disorders of the Central Nervous System and of the gastrointestinal tract, such as irritable intestine syndrome. Compounds with 5-HT₆ receptor affinity are also useful in the treatment of anxiety, depression and cognitive memory disorders [M. Yoshioka, et al., Ann. NY Acad. Sci., 1998, 861, 244; A. Bourson, et al., Br. J. Pharmacol. , 1998, 125, 1562; D.C. Rogers, et al., Br. J. Pharmacol. Suppl., 1999, 127, 22P; A. Bourson, et al., J. Pharmacol. Exp. Ther. , 1995, 274, 173; A.J. Sleight, et al., Behav. Brain Res. , 1996, 73, 245; T.A. Branchek, et al., nnu. Rev. Pharmacol. Toxicol. , 2000, 40, 319; C. Routledge, et al., Br. J. Pharmacol. , 2000, 130, 1606]. It has been shown that typical and atypical antipsychotic drugs for treating schizophrenia have a high affinity for 5-HT₆ receptors [B.L. Roth, et al., J. Pharmacol. Exp. Ther. , 1994, 268, 1403; C.E. Glatt, et al., Mol. Med. , 1995, 1, 398; F.J. Mosma, et al., Mol. Pharmacol. , 1993, 43, 320; T. Shinkai, et al., Am. J. Med. Genet. , 1999, 88, 120]. Compounds with 5-HT₆ receptor affinity are useful for treating infant hyperkinesia (ADHD, attention deficit / hyperactivity disorder) [W.D. Hirst, et al., Br. J. Pharmacol. , 2000, 130, 1597; C. Gérard, et al., Brain Research , 1997, 746, 207; M.R. Pranzatelli, Drugs of Today , 1997, 33, 379].

Moreover, it has been shown that the 5-HT₆ receptor also plays a role in food ingestion [Neuropharmacology, 2001, 41, 210-219]. In this sense, the 5-HT₆ receptor has generated enormous interest amongst academic and pharmaceutical industry scientists as a molecular target for the development of a new generation of safe and more effective anti-obesity drugs. Heal, D. et al. describe in Pharm. Ther.; 2008; 117(2), 207-31, the major developments that have occurred in the field of medicinal chemistry and pharmacology of 5-HT₆ ligands, with particular emphasis on their application as novel anti-obesity drugs. The last 5 years have witnessed an increasing understanding of the 5-HT₆ receptor and its structural requirements, producing an explosion in the number and diversity of novel, highly selective 5-HT₆ receptor agonists, partial agonists and antagonists that have been designed and synthesised.

Food ingestion disorders, particularly obesity, are a serious, fast growing threat to the health of humans of all age groups, since they increase the risk of developing other serious, even life-threatening diseases such as diabetes or coronary diseases as well.

There are several documents describing the use of sulphonamide derivatives exhibiting a modulating activity at 5-HT₆ receptor which have been useful in the treatment of feeding disorders like anorexia, obesity, bulimia and similar disorders and also type 2 diabetes (EP1747779, EP1676841, EP1632491, WO2003/042175, WO2007/004959, WO2006/126939). Particularly, the compound 5-chloro-*N*-[3-(2-(dimethylamino)ethyl)-1H-indol-5-yl] naphthalene-2-sulphonamide, also known as E-6837, has shown a selective and high affinity binding for the recombinant human 5-HT₆ receptor, producing a reduction in the food intake, thus inducing a significant body weight loss [Fisas, A. et al., Brit. J. Pharm.; 2006 ; 148 ; 973-983].

Therefore, it would be highly desirable to develop new compounds that are particularly suitable as active ingredients in medicaments, especially in medicaments for the prophylaxis and/or treatment of disorders or diseases related to 5-HT₆.

### SUMMARY OF THE INVENTION

The inventors have surprisingly found that the substituted *N*-imidazo[2,1-b]thiazole-5-sulfonamide compounds of general formula I given below show good to excellent affinity for 5-HT₆-receptors. These compounds are therefore particularly suitable as pharmacologically active agents in a medicament for the prophylaxis and/or treatment of disorders or diseases related to 5-HT₆-receptors.

In a first aspect, the present invention is directed to a *N*-imidazo[2,1-b]thiazole-5-sulfonamide compound of formula (I): 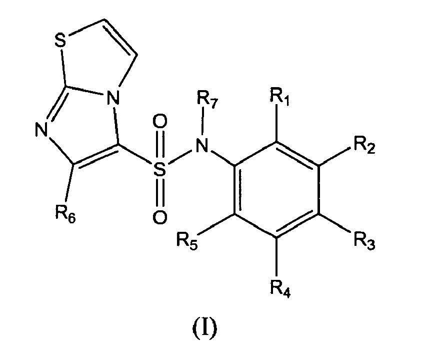 wherein:
R₁ to R₅ are independently selected from hydrogen; linear or branched C₁₋₅ alkyl radical optionally substituted by at least one halogen; linear or branched C₁₋₅ alkenyl radical, halogen, nitro, NR₈R₉, C₁₋₅ alkyl-N(R₈R₉) and OR₁₀, wherein R₈ and R₉ are independently selected from hydrogen, linear or branched C₁₋₅ alkyl radical or together with the nitrogen atom to which they are attached form an heterocyclic group, R₁₀ is a linear or branched C₁₋₅ alkyl radical, provided that at least one of R₁ to R₅ is not hydrogen, or
R₁ and R₂ or R₂ and R₃ or R₃ and R₄ or R₄ and R₅ form, together with the benzene ring to which they are attached, a substituted or unsubstituted 9 to 12-member cyclic system optionally containing 1, 2 or 3 heteroatoms selected from nitrogen, oxygen and sulphur atoms, wherein the cyclic formed by R₁ and R₂ or by R₂ and R₃ or by R₃ and R₄ or by R₄ and R₅ attached to the benzene ring, is substituted by at least one substituent independently selected from the group consisting of hydrogen; C₁₋₅ alkyl radical or C₁₋₅ alkenyl radical optionally substituted by at least one halogen, -OH, oxo, -N(R₁₁)(R₁₂), - O-C₁₋₅ alkyl or -S-C₁₋₅ alkyl; =O; OH; -C(O)R₁₀; -C(O)OR₁₀ and -N(R₁₁)(R₁₂), wherein R₁₀ is a linear or branched C₁₋₅ alkyl radical, R₁₁ and R₁₂ are independently selected from hydrogen and linear or branched C₁₋₅ alkyl radical, or
R₁ with R₂ and R₃, or R₂ with R₃ and R₄, or R₃ with R₄ and R₅ form, together with the benzene ring to which they are attached, a substituted or unsubstituted 11 to 13-member condensed polycyclic system optionally containing 1, 2 or 3 heteroatoms selected from nitrogen, oxygen and sulphur atoms,
R₆ is a halogen or a C₁₋₅ alkyl radical optionally substituted by at least one halogen,
R₇ is hydrogen, a linear or branched C₁₋₅ alkyl radical or a cycloalkyl group,
with the proviso that:
   - when R₁ and R₂, or R₂ and R₃, or R₃ and R₄, or R₄ and R₅ form, together with the benzene ring to which they are attached, the cyclic system: 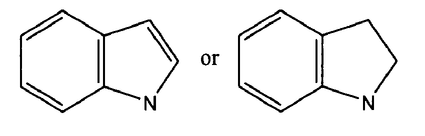 then pyrrol ring or dihydropyrrol ring is not fully substituted by hydrogen or is not substituted by only one group -N(R₁₁)(R₁₂) or by only one group C₁₋₅ alkyl-(NR₁₁)(R₁₂) or is not fused to another heterocyclyl group; and
   - when R₁ and R₂, or R₂ and R₃, or R₃ and R₄, or R₄ and R₅ form, together with the benzene ring to which they are attached, the cyclic system: 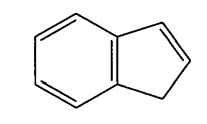 then the cyclopentadiene ring is not substituted by only one group - N(R₁₁)(R₁₂); and
   - R₁ and R₂, or R₂ and R₃, or R₃ and R₄, or R₄ and R₅ do not form, together with the benzene ring to which they are attached, the optionally substituted cyclic system: 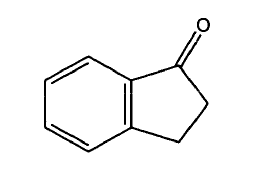
   - the compound: 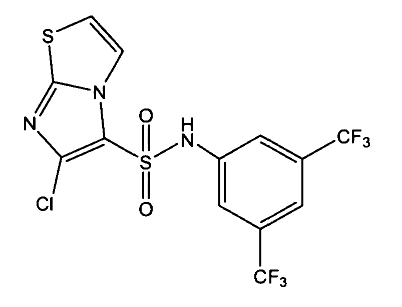 is not included in formula (I);
or a pharmaceutically acceptable salt, isomer or solvate thereof.

A second aspect of the present invention relates to a pharmaceutical composition comprising a compound of general formula (I) as defined above or a pharmaceutically acceptable salt, isomer or solvate thereof, and a pharmaceutically acceptable carrier, adjuvant or vehicle.

Another aspect of the present invention relates to a compound of general formula (I) as defined above or a pharmaceutically acceptable salt, isomer or solvate thereof for its use as a medicament.

Another aspect of the invention refers to a compound of the general formula (I) as defined above or a pharmaceutically acceptable salt, isomer or solvate thereof for its use as a medicament in the prophylaxis, treatment and/or improvement of a 5-HT₆ mediated disease or condition.

In a particular embodiment, the 5-HT₆ mediated disease or condition is selected from a disorder or a disease related to food intake; obesity; bulimia; anorexia; cachexia; type II diabetes; irritable colon syndrome; a disorder of the central nervous system; anxiety; panic attacks; depression; bipolar disorders; cognitive disorders; memory disorders; senile dementia; psychosis; neurodegenerative disorders; schizophrenia; psychosis; and hyperactivity disorders.

In a preferred embodiment, the disorder or disease related to food intake is the regulation of the appetite or the maintenance, increase or reduction of body weight.

In another preferred embodiment, the neurodegenerative disorder is selected from the group consisting of Morbus Alzheimer, Morbus Parkinson, Morbus Huntington and Multiple Sclerosis.

In another preferred embodiment, the hyperactivity disorder is an attention deficit.

A further aspect of the present invention relates to the use of a compound of general formula (I) as defined above in the manufacture of a medicament.

In an additional aspect the present invention is directed to the use of a compound of general formula (I) as defined above for the manufacture of a medicament for the prophylaxis and/or treatment of a 5-HT₆ mediated disease or condition.

### DETAILED DESCRIPTION OF THE INVENTION

In the definition of compounds of formula (I) the following terms have the meaning indicated:
"C₁₋₅ alkyl" refers to a linear or branched hydrocarbon chain radical consisting of carbon and hydrogen atoms, containing no insaturation, having one to five carbon atoms, and which is attached to the rest of the molecule by a single bond, e. g., methyl, ethyl, n-propyl, i-propyl, n-butyl, t-butyl, n-pentyl, etc.
"C₁₋₅ alkenyl" refers to a straight or branched hydrocarbon radical consisting of carbon and hydrogen atoms, having two to five carbon atoms and having one or more unsaturated bonds, e.g., ethenyl, propenyl, etc.
"9 to 12-member condensed cyclic system" refers to a stable 9- to 12 membered ring radical which consists of a benzene ring fused to a 5 to 8-member ring, said 5- to 8-member ring being a cycloalkyl, an aryl or a heterocyclyl radical.
"Cycloalkyl" refers to a stable 3-to 8-membered ring radical which is saturated or partially saturated, and which consists solely of carbon and hydrogen atoms, such as cyclohexyl or cyclopentyl.
"Aryl" refers to a stable 5-to 8-membered aromatic ring radical, and which consists solely of carbon and hydrogen atoms, such as phenyl or cyclooctatetraene.
"Heterocyclyl" refers to a stable 5-to 8 membered ring radical which consists of carbon atoms and from one to three heteroatoms selected from the group consisting of nitrogen, oxygen, and sulphur. For the purposes of this invention, the heterocycle may be partially or fully saturated or aromatic. It may be optionally fused to a cycloalkyl, aryl or heterocyclyl group as defined above. Examples of such heterocycles include, but are not limited to pyrrolidine, pyridine, thiophene, furan, etc.
"11 to 13-member condensed polycyclic system" refers to a stable 11- to 13-membered ring radical which consists of a benzene ring fused to a bicyclic radical which is saturated, partially saturated or aromatic, optionally containing 1, 2, or 3 heteroatoms selected from nitrogen, oxygen or sulphur atoms. Unless otherwise stated specifically in the specification, the bicyclic radical is optionally substituted with at least one substituent independently selected from the group consisting of hydrogen; C₁₋₅ alkyl radical or C₁₋₅ alkenyl radical optionally substituted by at least one halogen, -OH, oxo, -N(R₁₁)(R₁₂), -O-C₁₋₅ alkyl or -S-C₁₋₅ alkyl; =O; OH; -C(O)R₁₀; -C(O)OR₁₀ and - N(R₁₁)(R₁₂), wherein R₁₀ is a linear or branched C₁₋₅ alkyl radical, R₁₁ and R₁₂ are independently selected from hydrogen and linear or branched C₁₋₅ alkyl radical. Examples of this condensed polycyclic system are: 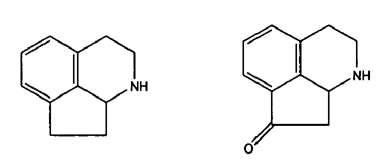
"Halogen" refers to bromo, chloro, iodo or fluoro.

In a particular embodiment of the invention, the substituents R₂ and R₃ of the compound of formula (I) form, together with the benzene ring to which they are attached, a 9 to 12-member cyclic system optionally containing 1, 2 or 3 heteroatoms selected from nitrogen, oxygen and sulphur atoms, wherein the cyclic formed by R₂ and R₃ attached to the benzene ring, is substituted with at least one substituent independently selected from the group consisting of hydrogen, C₁₋₅ alkyl radical optionally substituted by at least one substituent independently selected from the group consisting of hydrogen; C₁₋₅ alkyl radical or C₁₋₅ alkenyl radical optionally substituted by at least one halogen, -OH, oxo, -N(R₁₁)(R₁₂), -O-C₁₋₅ alkyl or -S-C₁₋₅ alkyl; =O; OH; -C(O)R₁₀; -C(O)OR₁₀ and -N(R₁₁)(R₁₂), wherein R₁₀ is a linear or branched C₁₋₅ alkyl radical, R₁₁ and R₁₂ are independently selected from hydrogen and linear or branched C₁₋₅ alkyl radical. In a preferred embodiment, the 9 to 12-member cyclic system contains 1 or 2 nitrogen atoms.

Ina another particular embodiment, the cyclic formed by R₂ and R₃ attached to the benzene ring, is substituted with at least one substituent independently selected from the group consisting of hydrogen, =O, OH, -C(O)R₁₀, -N(R₁₁)(R₁₂), C₁₋₅ alkyl-OH and C₁₋₅ alkyl-N(R₁₁)(R₁₂), wherein R₁₀ is a linear or branched C₁₋₅ alkyl radical, R₁₁ and R₁₂ are independently selected from hydrogen and linear or branched C₁₋₅ alkyl radical.

In a particular embodiment, R₆ is a halogen, preferably chloro or bromo.

In another particular embodiment, R₇ is hydrogen.

In another particular embodiment, R₄ is hydrogen.

Even, in another particular embodiment, R₁ and R₅ are hydrogen.

Particular individual compounds of the invention include the compounds 1-60 in the examples, either as salts or as free bases.

| **N°** | **STRUCTURE** | **Autonom** | **MS (APCI (M+H)⁺)** |
|---|---|---|---|
| 1 | 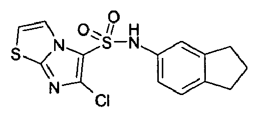 | 6-Chloro-imidazo[2,1-b] thiazole-5-sulfonic acid indan-5-ylamide | 354 |
| 2 | 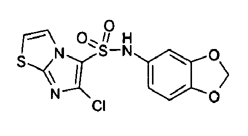 | 6-Chloro-imidazo[2,1-b] thiazole-5-sulfonic acid benzo[1,3]dioxol-5-ylamide | 358 |
| 3 | 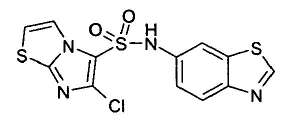 | 6-Chloro-imidazo[2,1-b] thiazole-5-sulfonic acid benzothiazol-6-ylamide | 371 |
| 4 | 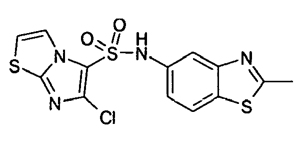 | 6-Chloro-imidazo[2,1-b] thiazole-5-sulfonic acid (2-methyl-benzothiazol-5-yl)-amide | 385 |
| 5 | 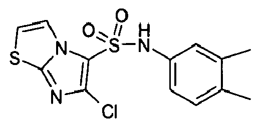 | 6-Chloro-imidazo[2,1-b] thiazole-5-sulfonic acid (3,4-dimethyl-phenyl)-amide | 342 |
| 6 | 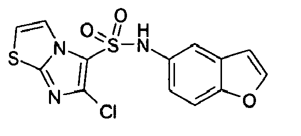 | 6-Chloro-imidazo[2,1-b] thiazole-5-sulfonic acid benzofuran-5-ylamide | 354 |
| 7 | 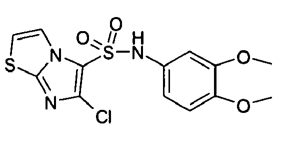 | 6-Chloro-imidazo[2,1-b] thiazole-5-sulfonic acid (3,4-dimethoxy-phenyl) -amide | 374 |
| 8 | 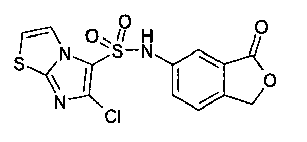 | 6-Chloro-imidazo[2,1-b] thiazole-5-sulfonic acid (3-oxo-1,3-dihydro-isobenzo furan-5-yl)-amide | 370 |
| 9 | 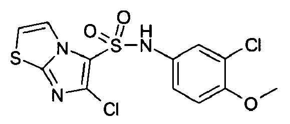 | 6-Chloro-imidazo[2,1-b] thiazole-5-sulfonic acid (3-chloro-4-methoxy-phenyl) -amide | 378 |
| 10 | 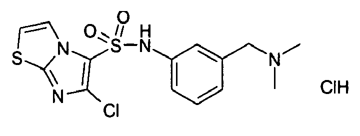 | 6-Chloro-imidazo[2,1-b] thiazole-5-sulfonic acid (3-dimethylaminomethyl-phenyl) -amide | 371 |
| 11 | 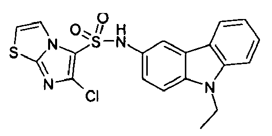 | 6-Chloro-imidazo[2,1-b] thiazole-5-sulfonic acid (9-ethyl-9H-carbazol-3-yl)-amide | 431 |
| 12 | 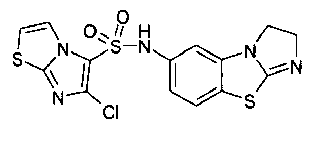 | 6-Chloro-imidazo[2,1-b] thiazole-5-sulfonic acid (2,3-dihydro-benzo[d]imidazo [2,1-b]thiazol-6-yl)-amide | 412 |
| 13 | 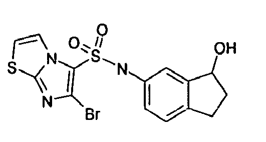 | 6-Bromo-imidazo[2,1-b]thiazole-5-su Ifonic acid (3-hydroxy-indan-5-yl)-amide | 415 |
| 14 | 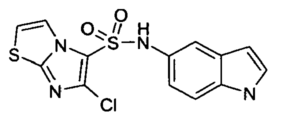 | 6-Chloro-imidazo[2,1-b] thiazole-5-sulfonic acid (1H-indol-5-yl)-amide | 353 |
| 15 | 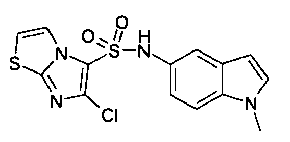 | 6-Chloro-imidazo[2,1-b] thiazole-5-sulfonic acid (1 methyl -1H-indol-5-y I)-amide | 367 |
| 16 | 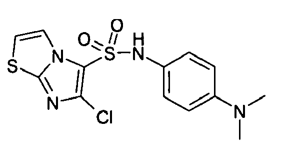 | 6-Chloro-imidazo[2,1-b] thiazole-5-sulfonic acid (4-dimethylamino-phenyl)- amide | 357 |
| 17 | 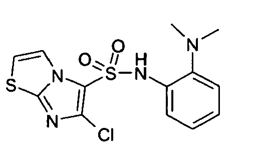 | 6-Chloro-imidazo[2,1-b] thiazole-5-sulfonic acid (2-dimethyl amino-phenyl)- amide | 357 |
| 18 | 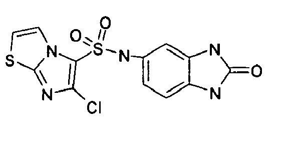 | 6-Chloro-imidazo[2,1-b]thiazole-5-s ulfonic acid (2-oxo-2,3-dihydro-1H-benzoimidazol-5-yl)-amide | 370 |
| 19 | 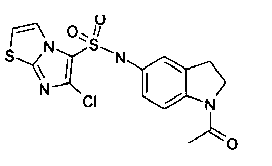 | 6-Chloro-imidazo[2,1-b]thiazole-5-s ulfonic acid (1-acetyl-2,3-dihydro-1 H-indol-5-yl)-amide | 397 |
| 20 | 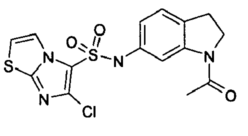 | 6-Chloro-imidazo[2,1-b]thiazole-5-s ulfonic acid (1-acetyl-2,3-dihydro-1H-indol-6-yl)-amide | 397 |
| 21 | 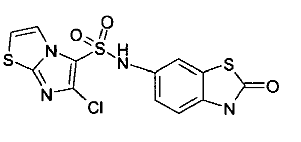 | 6-Chloro-imidazo[2,1-b]thiazole-5-s ulfonic acid (2-oxo-2,3-dihydro-ben zothiazol-6-yl)-amide | 387 |
| 22 | 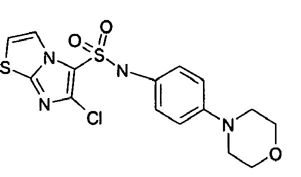 | 6-Chloro-imidazo[2,1-b]thiazole-5-s ulfonic acid (4-morpholin-4-yl-phen yl)-amide | 399 |
| 23 | 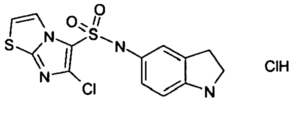 | 6-Chloro-imidazo[2,1-b]thiazole-5-s ulfonic acid (2,3-dihydro-1H-indol-5-yl)-amide hydrochloride | 355 |
| 24 | 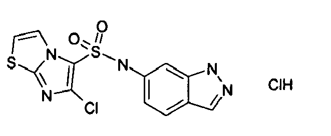 | 6-Chloro-imidazo[2,1-b]thiazole-5-s ulfonic acid (1H-indazol-6-yl)-amide hydrochloride | 354 |
| 25 | 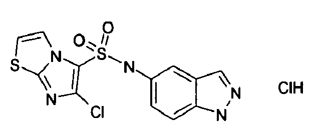 | 6-Chloro-imidazo[2,1-b]thiazole-5-s ulfonic acid (1H-indazol-5-yl)-amide hydrochloride | 354 |
| 26 | 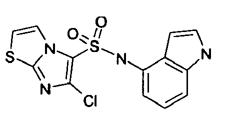 | 6-Chloro-imidazo[2,1-b]thiazole-5-s ulfonic acid (1H-indol-4-yl)-amide | 353 |
| 27 | 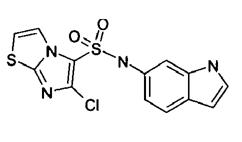 | 6-Chloro-imidazo[2,1-b]thiazole-5-s ulfonic acid (1H-indol-6-yl)-amide | 354 |
| 28 | 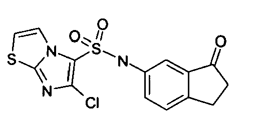 | 6-Chloro-imidazo[2,1-b]thiazole-5-sulfonic acid (3-oxo-indan-5-yl)-amide | 368 |
| 29 | 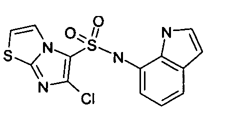 | 6-Chloro-imidazo[2,1-b]thiazole-5-sulfonic acid (1H-indol-7-yl)-amide | 353 |
| 30 | 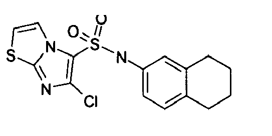 | 6-Chloro-imidazo[2,1-b]thiazole-5-sulfonic acid (5,6,7,8-tetrahydro-naphthalen-2-yl)-amide | 368 |
| 31 | 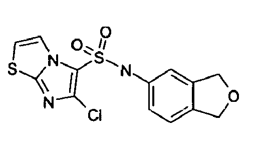 | 6-Chloro-imidazo[2,1-b]thiazole-5-sulfonic acid (1,3-dihydro-isobenzo furan-5-yl)-amide | 356 |
| 32 | 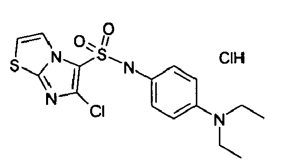 | 6-Chloro-imidazo[2,1-b]thiazole-5-sulfonic acid (4-diethylamino-phenyl) -amide hydrochloride | 385 |
| 33 | 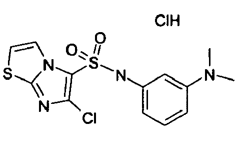 | 6-Chloro-imidazo[2,1-b]thiazole-5-sulfonic acid (3-dimethylamino-phenyl) -amide hydrochloride | 357 |
| 34 | 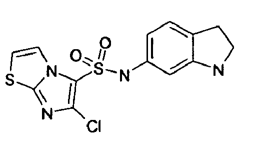 | 6-Chloro-imidazo[2,1-b]thiazole-5-sulfonic acid (2,3-dihydro-1H-indol-6-yl)-amide | 355 |
| 35 | 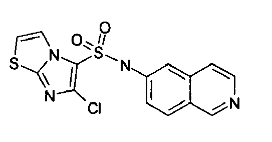 | 6-Chloro-imidazo[2,1-b]thiazole-5-sulfonic acid isoquinolin-6-ylamide | 365 |
| 36 | 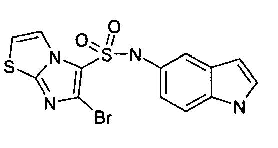 | 6-Bromo-imidazo[2,1-b]thiazole-5-sulfonic acid (1H-indol-5-yl)-amide | 397 |
| 37 | 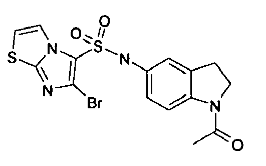 | 6-Bromo-imidazo[2,1-b]thiazole-5-sulfonic acid (1-acetyl-2,3-dihydro-1 H-indol-5-yl)-amide | 442 |
| 38 | 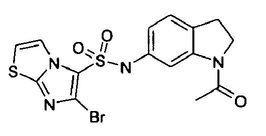 | 6-Bromo-imidazo[2,1-b]thiazole-5-su sulfonic acid (1-acetyl-2,3-dihydro-1 H-indol-6-yl)-amide | 442 |
| 39 | 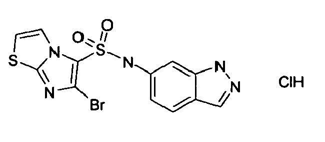 | 6-Bromo-imidazo[2,1-b]thiazole-5-sulfonic acid (1H-indazol-6-yl)-amide hydrochloride | 399 |
| 40 | 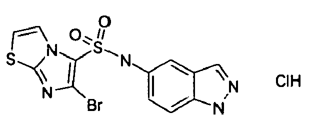 | 6-Bromo-imidazo[2,1-b]thiazole-5-sulfonic acid (1H-indazol-5-yl)-amide hydrochloride | 399 |
| 41 | 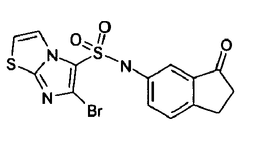 | 6-Bromo-imidazo[2,1-b]thiazole-5-sulfonic acid (3-oxo-indan-5-yl)-amide hydrochloride | 413 |
| 42 | 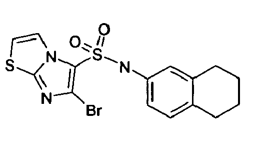 | 6-Bromo-imidazo[2,1-b]thiazole-5-sulfonic acid (5,6,7,8-tetrahydro-naphthalen-2-yl)-amide | 413 |
| 43 | 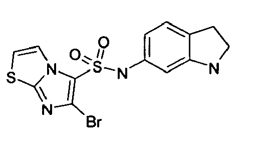 | 6-Bromo-imidazo[2,1-b]thiazole-5-sulfonic acid (2,3-dihydro-1H-indol-6 -yl)-amide | 400 |
| 44 | 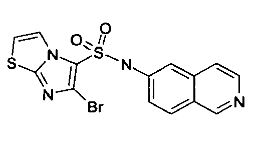 | 6-Bromo-imidazo[2,1-b]thiazole-5-sulfonic acid isoquinolin-6-ylamide hydrochloride | 410 |
| 45 | 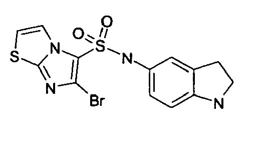 | 6-Bromo-imidazo[2,1-b]thiazole-5-su Ifonic acid (2,3-dihydro-1H-indol-5 -yl)-amide | 400 |
| 46 | 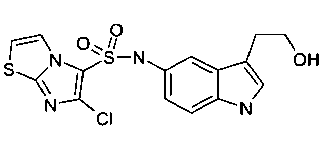 | 6-Chloro-imidazo[2,1-b]thiazole-5-s ulfonic acid [3-(2-hydroxy-ethyl)-1 H-indol-5-yl]-amide | 397 |
| 47 | 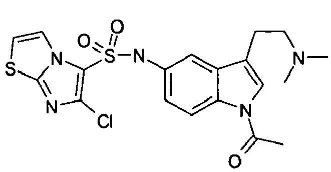 | 6-Chloro-imidazo[2,1-b]thiazole-5-s ulfonic acid [1-acetyl-3-(2-dimethy lamino-ethyl)-1H-indol-5-yl]-amide | 466 |
| 48 | 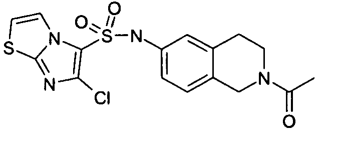 | 6-Chloro-imidazo[2,1-b]thiazole-5-s ulfonic acid (2-acetyl-1,2,3,4-tetr ahydro-isoquinolin-6-yl)-amide | 411 |
| 49 | 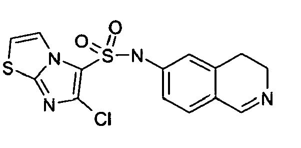 | 6-Chloro-imidazo[2,1-b]thiazole-5-s ulfonic acid (3,4-dihydro-isoquinol in-6-yl)-amide | 367 |
| 50 | 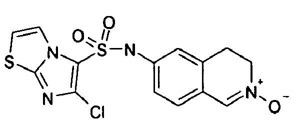 | 6-Chloro-imidazo[2,1-b]thiazole-5-s ulfonic acid (2-oxy-3,4-dihydro-iso quinolin-6-yl)-amide | 383 |
| 51 | 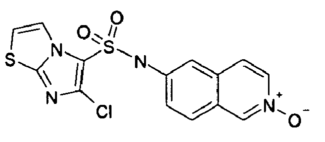 | 6-Chloro-imidazo[2,1-b]thiazole-5-s ulfonic acid (2-oxy-isoquinolin-6-y l)-amide | 381 |
| 52 | 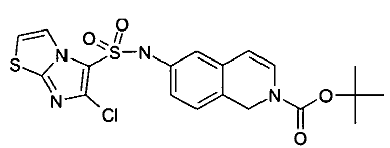 | 6-(6-Chloro-imidazo[2,1-b]thiazole-5-sulfonylamino)-1H-isoquinoline-2-carboxylic acid tert-butyl ester | 467 |
| 53 | 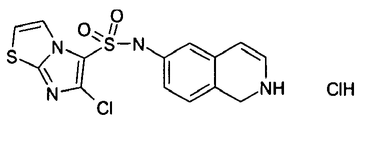 | 6-Chloro-imidazo[2,1-b]thiazole-5-s ulfonic acid (1,2-dihydro-isoquinol in-6-yl)-amide hydrochloride | 367 |
| 54 | 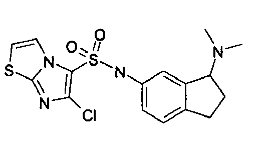 | 6-Chloro-imidazo[2,1-b]thiazole-5-s ulfonic acid (3-dimethylamino-indan -5-yl)-amide | 397 |
| 55 | 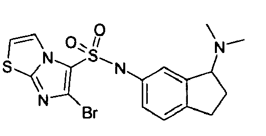 | 6-Bromo-imidazo[2,1-b]thiazole-5-su lfonic acid (3-dimethylamino-indan-5-yl)-amide | 442 |
| 56 | 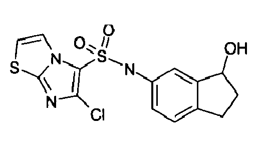 | 6-Chloro-imidazo[2,1-b]thiazole-5-s ulfonic acid (3-hydroxy-indan-5-yl) -amide | 370 |
| 57 | 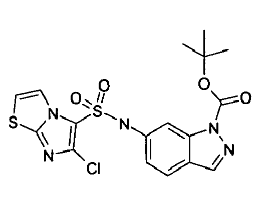 | 6-(6-Chloro-imidazo[2,1-b]thiazole-5-sulfonylamino)-indazole-1-carboxy lic acid tert-butyl ester | 454 |
| 58 | 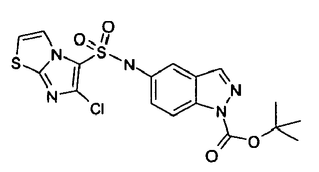 | 5-(6-Chloro-imidazo[2,1-b]thiazole-5-sulfonylamino)-indazole-1-carboxy lic acid tert-butyl ester | 454 |
| 59 | 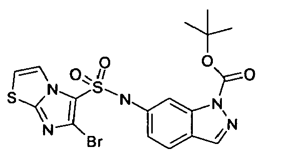 | 6-(6-Bromo-imidazo[2,1-b]thiazole-5 -sulfonylamino)-indazole-1-carboxyl ic acid tert-butyl ester | 499 |
| 60 | 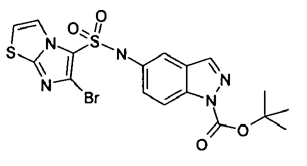 | 5-(6-Bromo-imidazo [2,1-b] thiazole-5-sulfonylamino)-indazole-1-carboxylic acid tert-butyl ester | 499 |

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by a ¹³C- or ¹⁴C-enriched carbon or ¹⁵N-enriched nitrogen are within the scope of this invention.

The term "pharmaceutically acceptable salts and solvates" refers to any pharmaceutically acceptable salt, ester, solvate, or any other compound which, upon administration to the recipient is capable of providing (directly or indirectly) a compound as described herein. However, it will be appreciated that non-pharmaceutically acceptable salts also fall within the scope of the invention since those may be useful in the preparation of pharmaceutically acceptable salts. The preparation of salts and solvates can be carried out by methods known in the art.

For instance, pharmaceutically acceptable salts of compounds provided herein are synthesized from the parent compound which contains a basic or acidic moiety by conventional chemical methods. Generally, such salts are, for example, prepared by reacting the free acid or base forms of these compounds with a stoichiometric amount of the appropriate base or acid in water or in an organic solvent or in a mixture of the two. Generally, nonaqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p-toluenesulphonate. Examples of the alkali addition salts include inorganic salts such as, for example, sodium, potassium, calcium, ammonium, magnesium, aluminium and lithium salts, and organic alkali salts such as, for example, ethylenediamine, ethanolamine, N,N-dialkylenethanolamine, triethanolamine, glucamine and basic aminoacids salts.

The compounds of the invention may be in crystalline form either as free compounds or as solvates and it is intended that both forms are within the scope of the present invention. Methods of solvation are generally known within the art. Suitable solvates are pharmaceutically acceptable solvates. In a particular embodiment the solvate is a hydrate.

The compounds of formula (I) or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I), or of its salts, solvates or prodrugs.

The compounds of the present invention represented by the above described formula (I) may include stereoisomers depending on the presence of chiral centres or isomers depending on the presence of multiple bonds (e.g. Z, E). The single isomers, enantiomers or diastereoisomers and mixtures thereof in any ratio fall within the scope of the present invention.

The compounds of formula (I) defined above can be obtained by available synthetic procedures. For example, they can be prepared by reaction of a compound of general formula (II): 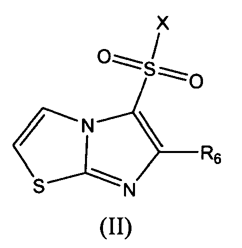 wherein:
X represents a leaving group, preferably a halogen atom, more preferably a chlorine atom, and
R₆ is a halogen or a C₁₋₅ alkyl radical optionally substituted by at least one halogen, with a compound of general formula (III), 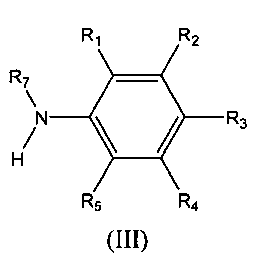
wherein:
R₁ to R₅ are as defined above, and R⁷ is hydrogen, C₁₋₅ alkyl radical or a cycloalkyl group_{.}

In a particular embodiment, the reaction is carried out in the presence of a solvent selected from the group consisting of acetonitrile, ethyle acetate, diethyl ether, *N,N*-dimethylformamide, pyridine, chloroform, dichloromethane, tetrahydrofurane, toluene and mixtures thereof.

Preferably, the reaction is carried out in the presence of at least one base, more preferably in the presence of at least one base selected from the group consisting of sodium hydrogen carbonate, potassium hydrogen carbonate, sodium carbonate, potassium carbonate, triethylamine, diisopropylethylamine and diethylisopropylamine.

The temperature of the reaction is preferably between 0 °C and 30 °C.

Compounds of general formula (III) are primary or secondary amines in most cases commercially available or may be prepared by processes known to those skilled in the art.

When the compound of general formula (III) is a primary amine (R₇ is hydrogen), the compound of general formula (I) obtained in the reaction is a secondary sulfonamide (Scheme 1).

When the compound of general formula (III) is a secondary amine (R₇ is not hydrogen), the compound of general formula (I) obtained in the reaction is a tertiary sulfonamide (Scheme 1).

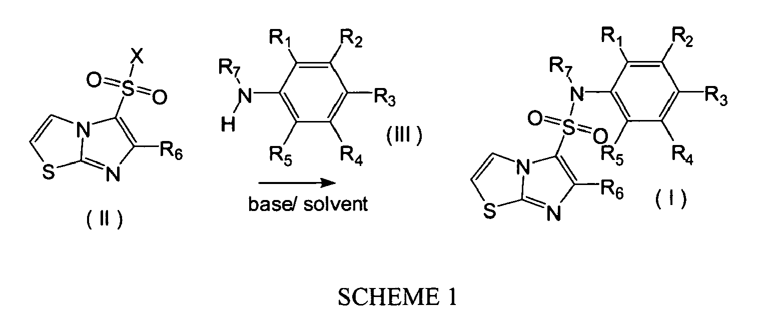

The tertiary sulfonamide may also be prepared by reaction of a secondary sulfonamide of general formula (I) wherein R₇ is hydrogen, with an alkyl or cycloalkyl halide, preferably an alkyl or cycloalkyl iodide (Scheme 2). Particularly, said reaction is made in a medium selected from the group consisting of acetonitrile, ethyle acetate, diethyl ether, *N,N*-dimethylformamide, pyridine, chloroform, dichloromethane, tetrahydrofurane, toluene and mixtures thereof. Said reaction is preferably carried out in the presence of at least one base, more preferably in the presence of at least one base selected from the group consisting of sodium hydrogen carbonate, potassium hydrogen carbonate, sodium carbonate, potassium carbonate.

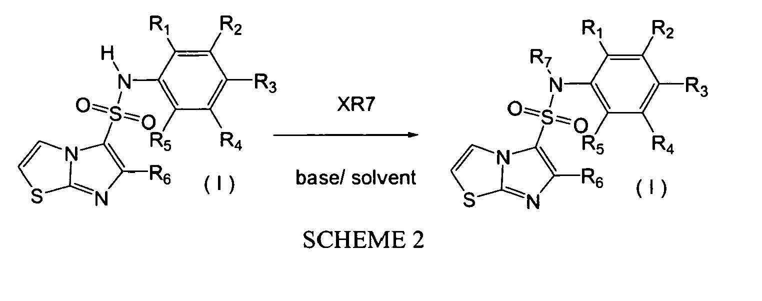

In the compounds of general formula (III), the protecting groups for the heteroatom may be used. Some examples include cyclic imide derivatives, such as maleimides or succinimides, a variety of carbamates, such as tert-butoxy-carbonyl (BOC) and fluorenylmethyloxycarbonyl (Fmoc), a variety of amides, such as acetamides, and alkyl and aryl amine derivatives, such as *N*-benzyl or *N*-allyl amines. Additional examples of nitrogen and oxygen protecting groups can be found in reference books such as Protective groups in Organic Chemistry, ed. J. F. W. McOmie, Plenum Press, 1973; and T.W. Greene & P.G.M. Wuts, Protective Groups in Organic Chemistry, John Wiley & sons, 1999.

When the deprotection process is necessary, the methodology is known to those skilled in the art.

If the compounds of general formula (I) are obtained in form of a mixture of stereoisomers, particularly enantiomers or diastereomers, said mixtures may be separated by standard procedures known to those skilled in the art, e.g. chromatographic methods or crystallization with chiral reagents.

The obtained reaction products may, if desired, be purified by conventional methods, such as crystallisation, chromatography and trituration. Where the above described processes for the preparation of compounds of the invention give rise to mixtures of stereoisomers, these isomers may be separated by conventional techniques such as preparative chromatography. If there are chiral centers the compounds may be prepared in racemic form, or individual enantiomers may be prepared either by enantiospecific synthesis or by resolution.

One preferred pharmaceutically acceptable form is the crystalline form, including such form in pharmaceutical composition. In the case of salts and solvates the additional ionic and solvent moieties must also be non-toxic. The compounds of the invention may present different polymorphic forms, it is intended that the invention encompasses all such forms.

The present invention further provides pharmaceutical compositions comprising a compound of general formula (I) as defined above, or a pharmaceutically acceptable salt, isomer or solvate thereof together with one or more pharmaceutically acceptable carrier, adjuvant or vehicle.

The term "carrier, adjuvant or vehicle" relates to molecular entities or substances with which the active ingredient is administered. Such pharmaceutical carriers, adjuvants or vehicles can be sterile liquids, such as waters and oils, including those of petroleum or with an animal, plant or synthetic origin, such as peanut oil, soybean oil, mineral oil, sesame oil and the like, excipients, disintegrants, wetting agents or diluents. Suitable pharmaceutical carriers are described in "Remington's Pharmaceutical Sciences" by E.W. Martin.

Examples of pharmaceutical compositions include any solid (tablets, pills, capsules, granules etc.) or liquid (solutions, suspensions or emulsions) composition for oral, topical or parenteral administration.

In a preferred embodiment the pharmaceutical compositions are in oral form, either solid or liquid. Suitable dose forms for oral administration may be tablets, capsules, syrops or solutions and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulfate.

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The pharmaceutical compositions may also be adapted for parenteral administration, such as sterile solutions, suspensions or lyophilized products in the appropriate unit dosage form. Adequate excipients can be used, such as bulking agents, buffering agents or surfactants.

The mentioned formulations will be prepared using standard methods such as those described or referred to in the Spanish and US Pharmacopoeias and similar reference texts.

Administration of the compounds or compositions of the present invention may be by any suitable method, such as intravenous infusion, oral preparations, and intraperitoneal and intravenous administration. Oral administration is preferred because of the convenience for the patient and the chronic character of the diseases to be treated.

Generally an effective administered amount of a compound of the invention will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.1 to 1000 mg/kg/day.

The compounds and compositions of this invention may be used with other drugs to provide a combination therapy. The other drugs may form part of the same composition, or be provided as a separate composition for administration at the same time or at different time.

Another aspect of this invention relates to a method of treating or preventing a 5-HT₆ mediated disease or condition, which method comprises administering to a patient in need of such a treatment a therapeutically effective amount of a compound of formula (I) or a pharmaceutical composition thereof. Among the 5-HT₆ mediated diseases that can be treated are: disorders or diseases related to food intake, preferably the regulation of appetite, the maintenance, increase or reduction of body weight; obesity; bulimia; anorexia; cachexia; type II diabetes; irritable colon syndrome; a disorder of the central nervous system; anxiety; panic attacks; depression; bipolar disorders; cognitive disorders; memory disorders; senile dementia; psychosis; neurodegenerative disorders, preferably selected from the group consisting of Morbus Alzheimer, Morbus Parkinson, Morbus Huntington and Multiple Sclerosis; schizophrenia; psychosis; and hyperactivity disorders, preferably attention deficit/hyperactivity disorder.

The following examples are given only as further illustration of the invention, they should not be taken as a definition of the limits of the invention.

### EXAMPLES

### Example 1. Synthesis of 6-Chloroimidazo [2,1-b]thiazole-5-sulfonyl chloride (compound of formula II).

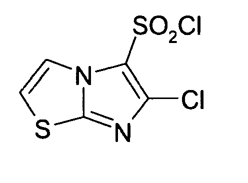

In a 1000 mL reaction flask, fitted with a mechanical stirrer, neat chlorosulfonic acid (3.60 mol, 240 mL) was placed and heated at 120 °C. (0.315 mol, 50 g ) 6-chloroimidazo [2,1-b]thiazole was added gradually to the chlorosulfonic acid. The reaction mixture was stirred at 120 °C for 2 h. The hydrogen chloride generated during sulfonation was trapped using a diluted sodium hydroxide solution.

The syrupy liquid was quenched slowly, with mechanical stirring into ice (3.50 kg). The decomposition of the excess chlorosulfonic acid should be carried out in a hood and the efficient gas absorption trap was used. The solid 6-chloroimidazo [2,1-b]thiazole-5-sulfonyl chloride separates was collected by filtration, washed with water and dried under vacuum. Obtained 49.82 g (62 % yield) white solid. This crude material may be used directly.
IR (KBr) 3159, 3141, 1426, 1380, 1271, 1188, 1144, 1092, 732, 622, 562 cm⁻¹.
¹HNMR (300 MHz, DMSO-*d*₆) δ ppm: 7,83 (d, *J*=4.5 Hz ,1 H), 7,34 (d, *J*=4.5 Hz ,1 H).
M.P.: 140-142 °C
[MH]⁺= 257.

### Example 2. Synthesis of 6-Bromoimidazo [2,1-b]thiazole-5-sulfonyl chloride (compound of formula II).

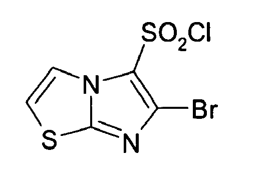

In a 25 mL flask, neat chlorosulfonic acid (30 mmol, 2.10 mL) was placed and heated at 120 °C. (2.5 mmol, 0.51 g ) 6-bromoimidazo [2,1-b]thiazole was added gradually to the chlorosulfonic acid. The reaction mixture was stirred at 120 °C for 2 h. The hydrogen chloride generated during sulfonation was trapped using a diluted sodium hydroxide solution.

The syrupy liquid was quenched slowly, with stirring into ice (30 g). The decomposition of the excess chlorosulfonic acid should be carried out in a hood and the efficient gas absorption trap was used. The solid 6-Bromoimidazo [2, 1-b]thiazole-5-sulfonyl chloride separates was collected by filtration, washed with water and dried under vacuum. Obtained 0.47 g (61% yield) white solid. This crude material may be used directly.
IR ( KBr) 3153, 3135, 1415, 1380,1342, 1255, 1193, 1144, 1089, 727, 575 cm⁻¹.
¹HNMR (300 MHz, DMSO-*d*₆) δ ppm: 7,85 (d, *J*=4.5 Hz ,1 H) , 7,34 (d, *J*=4.5 Hz ,1 H) [MH]⁺= 302
M.P.: 115-117 °C.

### Example 3: Synthesis of 6-Chloroimidazo [2,1-b]thiazole-5-sulfonic acid (1-acetyl-2,3-dihydro-1H-indol-6-yl)-amide (compound 20)

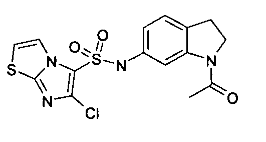

1-acetyl-6-amino-2,3-dihydro-(1H)-indole (176mg, 1 mmol), sodium hydrogen carbonate (235 mg, 2.8 mmol )were dissolved in 5 mL acetonitrile. The mixture is stirred 15 minutes, followed by the addition of a 6-chloro-imidazo[2,1-b]thiazole-5-sulfonyl chloride (270 mg, 1,05 mmol) . The reaction mixture was stirred at room temperature (18-22 °C) for 16 h., until complete conversion (TLC) CHCl3/MeOH 95:5. The insoluble resulting reaction mixture was removed by filtration and the solid was washed with acetonitrile (3mL) and (3 x 5 mL) water. The product remains insoluble was washed with dichloromethane and vacuum dried at 50°C, yielding 296 mg (76%) solid cream.
HPLC/MS 99,37 %
¹H NMR (300 MHz, DMSO-*d*₆) δ ppm: 2.07 (s, 3 H) 2.98 (t, 2 H) 4.00 (t, 2 H) 6.74 (d, 1 H) 7.04 (d, 1 H), 7.60 (d, 1 H), 7.81 (s, 1 H) 7.94 (d, 1 H) 10.70 (s, 1 H)
IR (KBr) 3128, 2883, 1625, 1589, 1488, 1493, 1345, 1319, 1257, 1140, 971, 670, 621 cm⁻¹.

### Example 4: Synthesis of 6-Chloroimidazo [2,1-b]thiazole-5-sulfonic acid(2,3-dihydro-1H-indol-5-yl)-amide hydrochloride (compound 23)

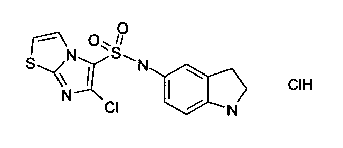

The 6-Chloroimidazo [2,1-b]thiazole-5-sulfonic acid (1-acetyl-2,3-dihydro-1H-indol-5-yl)-amide (160mg, 0,40 mmol) in 10 mL of hydrogen chloride 2,8 M solution (in ethanol) and 2 mL of water, the solution was refluxed for 16 h .The solvent was removed under reduced pressure and the residue solid was washed with ethyl acetate, vacuum dried at 50°C, yielding 137 mg (yield 88%).
HPLC/MS 95,6 %
¹H NMR (300 MHz, DMSO-*d*₆) δ ppm: 3.09 (t, *J*=7.69 Hz, 2 H) 3.63 (t, *J*=7.62 Hz, 2 H) 7.07 (d, *J*=8.35 Hz, 1 H) 7.17 (s, 1 H) 7.30 (d, *J*=8.50 Hz, 1 H) 7.64 (d, *J*=4.39 Hz, 1 H) 8.03 (d, *J*=4.39 Hz, 1 H) 11.26 (br. s., 1 H).

### Example 5: Synthesis of 6-Chloroimidazo [2,1-b]thiazole-5-sulfonic acid(3-oxo-indan-5-yl)-amide (compound 28)

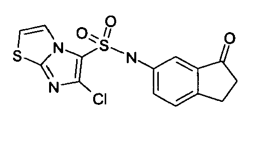

6-amino-indan-1-one (147 mg, 1 mmol), sodium hydrogen carbonate (235 mg, 2,8 mmol )were dissolved in 5 mL acetonitrile. The mixture is stirred 15 minutes, followed by the addition of a 6-chloro-imidazo[2,1-b]thiazole-5-sulfonyl chloride (270 mg, 1,05 mmol). The reaction mixture was stirred at room temperature (18-22 °C) for 16 h., until complete conversion (TLC) CHCl₃/MeOH 95:5. The solvent was removed under reduced pressure and the residue was taken up in ethyl acetate (25 mL) and washed with HCl IN (15 mL) and water (2x10 mL), dried and concentrated under reduced pressure to afforded, yielding 275 mg (yield 75%).
[MH]⁺= 368
¹H NMR (300 MHz, DMSO-*d*₆) δ ppm: 2.59 (d, *J*=5.86 Hz, 1 H) 2.56 (br. s., 1 H) 2.97 (d, *J*=5.57 Hz, 1 H) 7.29 (d, *J*=1.76 Hz, 1 H) 7.38 (d, *J*=1.90 Hz, 1 H) 7.43 - 7.55 (m, 1 H) 7.65 (d, *J*=4.39 Hz, 1 H) 7.98 (d, *J*=4.54 Hz, 1 H) 11.14 (s, 1 H).

### Example 6: Synthesis of 6-Bromoimidazo [2,1-b]thiazole-5-sulfonic acid(1H-indol-5-yl)-amide (compound 36)

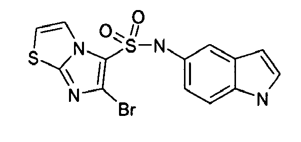

5-amino-indole (66 mg, 0.5 mmol), sodium hydrogen carbonate (126 mg, 1.5 mmol ) were dissolved in 5 mL acetonitrile. The mixture is stirred 15 minutes, followed by the addition of a 6-bromo-imidazo[2,1-b]thiazole-5-sulfonyl chloride (158 mg, 0.52 mmol). The reaction mixture was stirred at room temperature (18-22 °C) for 16 h., until complete conversion (TLC) CHCl₃/MeOH 95:5. The solvent was removed under reduced pressure and the residue was taken up in ethyl acetate (25 mL) and washed with HCl 1N (15 mL) and water (2x 10 mL), dried and concentrated under reduced pressure to afforded, yielding 152 mg (yield 76%).
[MH]⁺= 397
¹H NMR (300 MHz, DMSO-*d*₆) δ ppm: 6.31 (d, *J*=2.05 Hz, 1 H) 6.75 (dd, *J*=8.50, 2.05 Hz, 1 H) 7.16 - 7.24 (m, 2 H) 7.29 (t, *J*=2.64 Hz, 1 H) 7.51 (d, *J*=4.54 Hz, 1 H) 7.83 (d, *J*=4.54 Hz, 1 H) 10.37 (br. s., 1 H) 11.07 (br. s., 1 H).

### Pharmacological Methods:

### Binding to serotonin receptor 5ht₆

Cell membranes of HEK-293 cells expressing the 5HT₆-human recombinant receptor were supplied by Receptor Biology. In said membranes the receptor concentration is 2.18 pmol/mg protein and the protein concentration is 9.17 mg/ml. The experimental protocol follows the method of B. L. Roth et al. [B. L. Roth et al., The Journal of Pharmacology and Experimental Therapeutics, 1994, 268, 1403] with the following slight changes. The respective part of the literature description is hereby incorporated by reference and forms part of the disclosure.

The commercial membrane is diluted (1:40 dilution) with the binding buffer: 50 mM Tris-HCl, 10 mM MgCl₂, 0.5 mM EDTA (pH 7.4). The radioligand used is [³H]-LSD at a concentration of 2.7 nM with a final volume of 200 µl. incubation is initiated by adding 100 µl of membrane suspension, (≈22.9 µg membrane protein), and is prolonged for 60 minutes at a temperature of 37 °C. The incubation is ended by fast filtration in a Brandel Cell Harvester through fiber glass filters made by Schleicher & Schuell GF 3362 pretreated with a solution of polyethylenimine at 0.5 %. The filters are washed three times with three milliliters of buffer Tris-HCl 50 mM pH 7.4. The filters are transferred to flasks and 5 ml of Ecoscint H liquid scintillation cocktail are added to each flask. The flasks are allowed to reach equilibrium for several hours before counting with a Wallac Winspectral 1414 scintillation counter. Non-specific binding is determined in the presence of 100 µM of serotonin. Tests were made in triplicate. The inhibition constants (Kᵢ, nM) were calculated by non-linear regression analysis using the program EBDA/LIGAND described in Munson and Rodbard, Analytical Biochemistry, 1980, 107, 220, which is hereby incorporated by reference and forms part of the disclosure.

The binding results for some of the compounds of the invention are given in the following table:

| Compound | **5-HT₆ Ki (nM)** |
|---|---|
| 1 | 11.1 |
| 3 | 12.7 |
| 4 | 14.5 |
| 15 | 23 |
| 16 | 8.7 |
| 19 | 1.6 |
| 20 | 12.8 |
| 23 | 20 |
| 25 | 5.7 |
| 28 | 7.1 |
| 30 | 19 |
| 31 | 68 |
| 32 | 5.4 |
| 34 | 7.4 |
| 36 | 10 |
| 37 | 1.3 |
| 38 | 37.2 |
| 41 | 4.9 |
| 60 | 6.6 |

## Claims

1. A compound of formula (I): 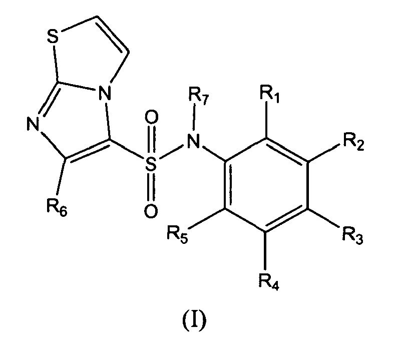 wherein:
R₁ to R₅ are independently selected from hydrogen; linear or branched C₁₋₅ alkyl radical optionally substituted by at least one halogen; linear or branched C₁₋₅ alkenyl radical, halogen, nitro, NR₈R₉, C₁₋₅ alkyl-N(R₈R₉) and OR₁₀, wherein R₈ and R₉ are independently selected from hydrogen, C₁₋₅ linear or branched alkyl radical or together with the nitrogen atom to which they are attached form an heterocyclic group, R₁₀ is a linear or branched C₁₋₅ alkyl radical, provided that at least one of R₁ to R₅ is not hydrogen, or
R₁ and R₂ or R₂ and R₃ or R₃ and R₄ or R₄ and R₅ form, together with the benzene ring to which they are attached, a substituted or unsubstituted 9 to 12-member condensed cyclic system optionally containing 1, 2 or 3 heteroatoms selected from nitrogen, oxygen and sulphur atoms, wherein the cyclic formed by R₁ and R₂ or by R₂ and R₃ or by R₃ and R₄ or by R₄ and R₅ attached to the benzene ring, is substituted with at least one substituent independently selected from the group consisting of hydrogen; C₁₋₅ alkyl radical or C₁₋₅ alkenyl radical optionally substituted by at least one halogen, -OH, oxo, - N(R₁₁)(R₁₂), -O-C₁₋₅ alkyl or -S-C₁₋₅ alkyl; =O; OH; -C(O)R₁₀; -C(O)OR₁₀ and - N(R₁₁)(R₁₂), wherein R₁₀ is a linear or branched C₁₋₅ alkyl radical, R₁₁ and R₁₂ are independently selected from hydrogen and linear or branched C₁₋₅ alkyl radical, or
R₁ with R₂ and R₃, or R₂ with R₃ and R₄, or R₃ with R₄ and R₅ form, together with the benzene ring to which they are attached, a substituted or unsubstituted 11 to 13-member condensed polycyclic system optionally containing 1, 2 or 3 heteroatoms selected from nitrogen, oxygen and sulphur atoms,
R₆ is selected from a halogen and a C₁₋₅ alkyl radical optionally substituted by at least one halogen,
R₇ is selected from hydrogen, a linear or branched C₁₋₅ alkyl radical and a cycloalkyl group,
with the proviso that:
- when R₁ and R₂, or R₂ and R₃, or R₃ and R₄, or R₄ and R₅ form, together with the benzene ring to which they are attached, the cyclic system: 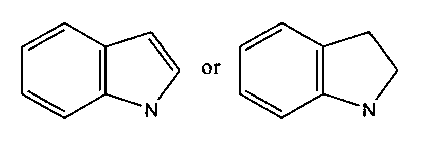 then pyrrol ring or dihydropyrrol ring is not fully substituted by hydrogen or is not substituted by only one group -N(R₁₁)(R₁₂) or by only one group C₁₋₅ alkyl-(NR₁₁)(R₁₂) or is not fused to another heterocyclyl group; and
- when R₁ and R₂, or R₂ and R₃, or R₃ and R₄, or R₄ and R₅ form, together with the benzene ring to which they are attached, the cyclic system: 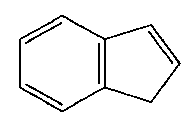 then the cyclopentadiene ring is not substituted by only one group - N(R₁₁)(R₁₂); and
- R₁ and R₂, or R₂ and R₃, or R₃ and R₄, or R₄ and R₅ do not form, together with the benzene ring to which they are attached, the optionally substituted cyclic system: 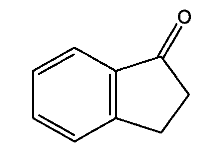
- the compound: 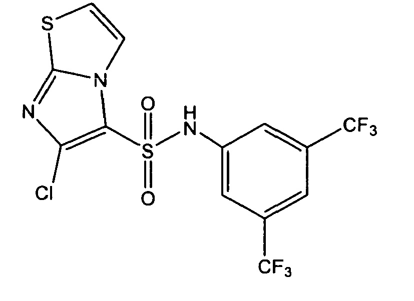 is not included in formula (I);
or a pharmaceutically acceptable salt, isomer or solvate thereof.

2. The compound according to claim 1 wherein the substituents R₂ and R₃ of the compound of formula (I) form, together with the benzene ring to which they are attached, a 9 to 12-member condensed cyclic system optionally containing 1, 2 or 3 heteroatoms selected from nitrogen, oxygen and sulphur atoms, wherein the cyclic formed by R₂ and R₃ attached to the benzene ring, is substituted with at least one substituent independently selected from the group consisting of hydrogen; C₁₋₅ alkyl radical or C₁₋₅ alkenyl radical optionally substituted by at least one halogen, -OH, oxo, - N(R₁₁)(R₁₂), -O-C₁₋₅ alkyl or -S-C₁₋₅ alkyl; =O; OH; -C(O)R₁₀; -C(O)OR₁₀ and - N(R₁₁)(R₁₂), wherein R₁₀ is a linear or branched C₁₋₅ alkyl radical, R₁₁ and R₁₂ are independently selected from hydrogen and linear or branched C₁₋₅ alkyl radical.

3. The compound according to claims 1 or 2 wherein the cyclic formed by R₂ and R₃ attached to the benzene ring, is substituted with at least one substituent independently selected from the group consisting of hydrogen, =O, OH, -C(O)R₁₀, -N(R₁₁)(R₁₂), C₁₋₅ alkyl-OH and C₁₋₅ alkyl-N(R₁₁)(R₁₂), wherein R₁₀ is a linear or branched C₁₋₅ alkyl radical, R₁₁ and R₁₂ are independently selected from hydrogen and linear or branched C₁₋₅ alkyl radical.

4. The compound according to any of claim 1 to 3 wherein R₆ is a halogen, preferably chloro or bromo.

5. The compound according to any of claims 1 to 4 wherein R₇ is hydrogen.

6. The compound according to any of claims 1 to 5 wherein R₄ is hydrogen.
The compound according to any of claims 1 to 6 wherein R₁ and R₅ are hydrogen.

7. A compound selected from:
1) 6-Chloro-imidazo [2,1-b] thiazole-5-sulfonic acid indan-5-ylamide;
2) 6-Chloro-imidazo [2,1-b] thiazole-5-sulfonic acid benzo [1,3] dioxol-5-ylamide;
3) 6-Chloro-imidazo [2,1-b] thiazole-5-sulfonic acid benzothiazol-6-yl-amide;
4) 6-Chloro-imidazo [2,1-b] thiazole-5-sulfonic acid (2-methyl-benzothiazol-5-yl)- amide;
5) 6-Chloro-imidazo [2,1-b] thiazole-5-sulfonic acid (3,4-dimethyl-phenyl)-amide;
6) 6-Chloro-imidazo [2,1-b] thiazole-5-sulfonic acid benzofuran-5-yl-amide;
7) 6-Chloro-imidazo [2,1-b] thiazole-5-sulfonic acid (3,4-dimethoxy-phenyl)-amide;
8) 6-Chloro-imidazo [2,1-b] thiazole-5-sulfonic acid (3-oxo-1,3-dihydro-isobenzo furan-5-yl)-amide;
9) 6-Chloro-imidazo [2,1-b] thiazole-5-sulfonic acid (3-chloro-4-methoxyphenyl)-amide;
10)6-Chloro-imidazo [2,1-b] thiazole-5-sulfonic acid (3-dimethylaminomethyl-phenyl)-amide hydrochloride;
11)6-Chloro-imidazo [2,1-b] thiazole-5-sulfonic acid (9-ethyl-9H-carbazol-3-yl)-amide;
12) 6-Chloro-imidazo [2,1-b] thiazole-5-sulfonic acid (2,3-dihydro-benzo[d]imidazo [2,1-b]thiazol-6-yl)-amide;
13)6-Bromo-imidazo [2,1-b] thiazole-5-sulfonic acid (3-hydroxy-indan-5-yl)-amide;
14) 6-Chloro-imidazo [2,1-b] thiazole-5-sulfonic acid (1H-indol-5-yl)-amide;
15)6-Chloro-imidazo [2,1-b] thiazole-5-sulfonic acid (1methyl-1H-indol-5-yl)-amide;
16) 6-Chloro-imidazo [2,1-b] thiazole-5-sulfonic acid (4-dimethylamino-phenyl)-amide;
17)6-Chloro-imidazo [2,1-b] thiazole-5-sulfonic acid (2-dimethyl amino-phenyl)-amide;
18)6-Chloro-imidazo [2,1-b] thiazole-5-sulfonic acid (2-oxo-2,3-dihydro-1H-benzoimidazol-5-yl)-amide;
19)6-Chloro-imidazo [2,1-b] thiazole-5-sulfonic acid (1-acetyl-2,3-dihydro-1H-indol-5-yl)-amide;
20)6-Chloro-imidazo [2,1-b] thiazole-5-sulfonic acid (1-acetyl-2,3-dihydro-1H-indol-6-yl)-amide;
21)6-Chloro-imidazo [2,1-b] thiazole-5-sulfonic acid (2-oxo-2,3-dihydro-benzothiazol-6-yl)-amide;
22) 6-Chloro-imidazo [2,1-b] thiazole-5-sulfonic acid (4-mopholin-4-yl-phenyl)-amide;
23)6-Chloro-imidazo [2,1-b] thiazole-5-sulfonic acid (2,3-dihydro-1H-indol-5-yl)-amide hydrochloride;
24)6-Chloro-imidazo [2,1-b] thiazole-5-sulfonic acid (1H-indazol-6-yl)-amide hydrochloride;
25)6-Chloro-imidazo [2,1-b] thiazole-5-sulfonic acid (1H-indazol-5-yl)-amide hydrochloride;
26) 6-Chloro-imidazo [2,1-b] thiazole-5-sulfonic acid (1H-indol-4-yl)-amide;
27) 6-Chloro-imidazo [2,1-b] thiazole-5-sulfonic acid (1H-indol-6-yl)-amide;
28) 6-Chloro-imidazo [2,1-b] thiazole-5-sulfonic acid (3-oxo-indan-5-yl)-amide;
29) 6-Chloro-imidazo [2,1-b] thiazole-5-sulfonic acid (1H-indol-7-yl)-amide;
30)6-Chloro-imidazo [2,1-b] thiazole-5-sulfonic acid (5,6,7,8-tetrahydronaphthalen-2-yl)-amide;
31) 6-Chloro-imidazo [2,1-b] thiazole-5-sulfonic acid (1,3-dihydro-isobenzofuran-5-yl)-amide;
32)6-Chloro-imidazo [2,1-b] thiazole-5-sulfonic acid (4-diethylamino-phenyl)-amide hydrochloride;
33) 6-Chloro-imidazo [2,1-b] thiazole-5-sulfonic acid (3-dimethylamino-phenyl)-amide hydrochloride;
34)6-Chloro-imidazo [2,1-b] thiazole-5-sulfonic acid (2,3-dihydro-1H-indol-6-yl)-amide;
35) 6-Chloro-imidazo [2,1-b] thiazole-5-sulfonic acid isoquinolin-6-yl-amide;
36) 6-Bromo-imidazo [2,1-b] thiazole-5-sulfonic acid (1H-indol-5-yl)-amide;
37)6-Bromo-imidazo [2,1-b] thiazole-5-sulfonic acid (1-acetyl-2,3-dihydro-1H-indol-5-yl)-amide;
38)6-Bromo-imidazo [2,1-b] thiazole-5-sulfonic acid (1-acetyl-2,3-dihydro-1H-indol-6-yl)-amide;
39)6-Bromo-imidazo [2,1-b] thiazole-5-sulfonic acid (1H-indazol-6-yl)-amide hydrochloride;
40)6-Bromo-imidazo [2,1-b] thiazole-5-sulfonic acid (1H-indazol-5-yl)-amide hydrochloride;
41)6-Bromo-imidazo [2,1-b] thiazole-5-sulfonic acid (3-oxo-indan-5-yl)-amide;
42) 6-Bromo-imidazo [2,1-b] thiazole-5-sulfonic acid (5,6,7,8-tetrahydronaphthalen-2-yl)-amide;
43)6-Bromo-imidazo [2,1-b] thiazole-5-sulfonic acid (2,3-dihydro-1H-indol-6-yl)-amide;
44) 6-Bromo-imidazo [2,1-b] thiazole-5-sulfonic acid isoquinolin-6-yl-amide;
45)6-Bromo-imidazo [2,1-b] thiazole-5-sulfonic acid (2,3-dihydro-1H-indol-5-yl)-amide;
46) 6-Chloro-imidazo [2,1-b] thiazole-5-sulfonic acid [3-(2-hydroxy-ethyl)-1H-indol-5-yl]-amide;
47) 6-Chloro-imidazo [2,1-b] thiazole-5-sulfonic acid [1-acetyl-3-(2-dimethylamino-ethyl)-1H-indol-5-yl]-amide;
48) 6-Chloro-imidazo [2,1-b] thiazole-5-sulfonic acid (2-acetyl-1,2,3,4-tetrahydro-isoquinolin-6-yl)-amide;
49) 6-Chloro-imidazo [2,1-b] thiazole-5-sulfonic acid (3,4-dihydro-isoquinolin-6-yl)-amide;
50) 6-Chloro-imidazo [2,1-b] thiazole-5-sulfonic acid (2-oxy-3,4-dihydro-isoquinolin-6-yl)-amide;
51)6-Chloro-imidazo [2,1-b] thiazole-5-sulfonic acid (2-oxy-isoquinolin-6-yl)-amide;
52) 6-(6-Chloro-imidazo [2,1-b] thiazole-5-sulfonylamino)-1H-isoquinoline-2-carboxylic acid tert-butyl ester;
53) 6-Chloro-imidazo [2,1-b] thiazole-5-sulfonic acid (1,2-dihydro-isoquinolin-6-yl)-amide hydrochloride;
54) 6-Chloro-imidazo [2,1-b] thiazole-5-sulfonic acid (3-dimethylamino-indan-5-yl)-amide;
55)6-Bromo-imidazo [2,1-b] thiazole-5-sulfonic acid (3-dimethylamino-indan-5-yl)-amide;
56)6-Chloro-imidazo [2,1-b] thiazole-5-sulfonic acid (3-hydroxy-indan-5-yl)-amide;
57) 6-(6-Chloro-imidazo [2,1-b] thiazole-5-sulfonylamino)-indazole-1-carboxylic acid tert-butyl ester;
58) 5-(6-Chloro-imidazo [2,1-b] thiazole-5-sulfonylamino)-indazole-1-carboxylic acid tert-butyl ester;
59) 6-(6-Bromo-imidazo [2,1-b] thiazole-5-sulfonylamino)-indazole-1-carboxylic acid tert-butyl ester;
60) 5-(6-Bromo-imidazo [2,1-b] thiazole-5-sulfonylamino)-indazole-1-carboxylic acid tert-butyl ester.

8. A pharmaceutical composition comprising a compound of general formula (I) as defined in any of claims 1 to 7 or a pharmaceutically acceptable salt, isomer or solvate thereof, and a pharmaceutically acceptable carrier, adjuvant or vehicle.

9. A compound of general formula (I) as defined in any of claims 1 to 7 or a pharmaceutically acceptable salt, isomer or solvate thereof for its use as a medicament.

10. A compound of the general formula (I) as defined in any of claims 1 to 7 or a pharmaceutically acceptable salt, isomer or solvate thereof for its use as a medicament in the prophylaxis, treatment and/or improvement of a 5-HT₆ mediated disease or condition.

11. The compound according to claim 10, wherein the 5-HT₆ mediated disease or condition is selected from a disorder or a disease related to food intake; obesity; bulimia; anorexia; cachexia; type II diabetes; irritable colon syndrome; a disorder of the central nervous system; anxiety; panic attacks; depression; bipolar disorders; cognitive disorders; memory disorders; senile dementia; psychosis; neurodegenerative disorders; schizophrenia; psychosis; and hyperactivity disorders.

12. The compound according to claim 11 wherein the disorder or disease related to food intake is the regulation of the appetite or the maintenance, increase or reduction of body weight.

13. The compound according to claim 11 wherein the neurodegenerative disorder is selected from the group consisting of Morbus Alzheimer, Morbus Parkinson, Morbus Huntington and Multiple Sclerosis.

14. The compound according to claim 11 wherein the hyperactivity disorder is an attention deficit.
